# EUROPEAN PATENT APPLICATION

(11) **EP 4 116 412 A1**
(43) Date of publication of application: **11.01.2023**
(21) Application number: 21763535.8
(22) Date of filing: 04.03.2021
(51) Int. Cl.: C12N 5/071, A61K 35/33, A61K 38/22, A61P 7/06, A61P 13/12

(54) **FIBROBLAST HAVING ENHANCED ERYTHROPOIETIN PRODUCTION ABILITY**

(30) Priority: 04.03.2020 JP 2020036421
(71) Applicant: Metcela Inc., Tsuruoka-shi, Yamagata, 997-0002 (JP)
(72) Inventor: IWAMIYA, Takahiro, Tsuruoka-shi, Yamagata 997-0002 (JP)
(74) Representative: Jones, Nicholas Andrew
(86) International application number: PCT/JP2021/008325
(87) International publication number: WO 2021/177387

(57) **Abstract**

It was found that, by culturing human fibroblasts using a medium supplemented with TNF-α and IL-4, EPO productivity in the fibroblasts can be improved. As a result, fibroblasts having enhanced EPO productivity were found, and means for improving a state where EPO productivity is decreased, and means for treating renal disorder or renal anemia, using the cells were discovered.

## Description

### TECHNICAL FIELD

The present invention relates to a fibroblast having enhanced erythropoietin (hereinafter also referred to as EPO) productivity. The present invention also relates to a production method for the fibroblast, and a pharmaceutical composition comprising the fibroblast.

### BACKGROUND ART

EPO is a hormone that contributes to promotion of erythrocyte production. EPO is mostly produced in the kidney, and the produced EPO acts on erythroblastic precursor cells in bone marrow, to play a role in promotion of erythrocyte production (differentiation and proliferation).

In recent years, the erythrocyte production-promoting ability of EPO is drawing attention, and effectiveness of EPO for kidney diseases such as renal anemia has been revealed (Non-patent Document 1). Thus, EPO preparations are widely used as therapeutic agents for renal anemia in actual clinical sites.

EPO is also known to have anti-inflammatory, antioxidative, antiapoptotic, and angiogenic promoting actions in various tissues. It has also been reported that administration of EPO to a subject having a renal disorder (acute kidney injury (AKI), chronic kidney disease (CKD), diabetic nephropathy, or the like) provides a kidney protection function that protects the kidney against a decline in renal function caused by the renal disorder (Non-patent Documents 2 and 3).

Further, the present inventors revealed that fibroblasts expressing vascular cell adhesion molecule-1 (VCAM-1, CD106) are useful as a cardiac cell culture material (Patent Document 1), and that cardiac fibroblasts expressing VCAM-1 are useful as an injectable composition for the purpose of treatment of a heart disease (Patent Document 2). However, usefulness of VCAM-1-expressing fibroblasts for purposes other than the use as a cardiac cell culture material, and usefulness of an injectable composition containing VCAM-1-expressing fibroblasts for diseases other than heart diseases, have been unclear.

### PRIOR ART DOCUMENTS

### [Patent Documents]

[Patent Document 1] WO 2016/006262
[Patent Document 2] WO 2018/155651

### [Non-patent Documents]

[Non-patent Document 1] Nephron, 1997; 77(2): 176-185
[Non-patent Document 2] Proc Natl Acad Sci., 2004; 101(41): 14907-14912
[Non-patent Document 3] J Renal Inj Prev., 2013; 2(1): 29-30

### SUMMARY OF THE INVENTION

### PROBLEMS TO BE SOLVED BY THE INVENTION

An object of the present invention is to provide fibroblasts having enhanced EPO productivity, and means for improving a state where EPO productivity is decreased, by use of the fibroblasts.

### MEANS FOR SOLVING THE PROBLEMS

In order to solve the above problems, the present inventors studied to discover that, by culturing human fibroblasts using a medium supplemented with TNF-α and IL-4, fibroblasts having enhanced EPO productivity can be obtained, thereby completing the present invention.

More specifically, the present invention includes the following first aspect (Invention A).
(A1) A fibroblast having enhanced EPO productivity.
(A2) The fibroblast according to (A1), which is positive for CD106.
(A3) The fibroblast according to (A1) or (A2), wherein the fibroblast is a kidney fibroblast.
(A4) The fibroblast according to any one of (A1) to (A3), wherein the EPO production is not less than 1.65 times higher than in a control fibroblast.
(A5) A cell population of fibroblast comprising the fibroblast according to any one of (Al) to (A4).
(A6) The cell population according to (A5), wherein the ratio of the CD106-positive fibroblast (in terms of the cell number) is not less than 4.7% relative to the total fibroblasts contained in the cell population.

The present invention also includes the following second aspect (Invention B).
(B1) A method of producing fibroblasts having enhanced EPO productivity, the method comprising:
   bringing fibroblasts into contact with TNF-α and IL-4; and
   culturing, under conditions allowing enhancement of EPO productivity, the fibroblasts that have been brought into contact.
(B2) The method according to (B1), wherein the fibroblasts having enhanced EPO productivity are positive for CD106.
(B3) The method according to (B2), further comprising selecting or concentrating CD106-positive fibroblasts using an anti-CD106 antibody.
(B4) The method according to any one of (B1) to (B3), wherein the fibroblast is a kidney fibroblast.
(B5) A fibroblast having enhanced EPO productivity, obtained by the production method according to any one of (B1) to (B4).
(B6) A cell population of fibroblast comprising the fibroblast according to (B5).

The present invention also includes the following third aspect (Invention C).
(C1) A pharmaceutical composition comprising: the fibroblast according to any one of (A1) to (A4) and (B5); or the cell population according to any one of (A5), (A6), and (B6).
(C2) The pharmaceutical composition according to (C1), for improvement of a state where EPO productivity is decreased.
(C3) The pharmaceutical composition according to (C1), for administration to a patient having a renal disorder.
(C4) The pharmaceutical composition according to (C3), wherein the renal disorder is chronic kidney disease (CKD).
(C5) The pharmaceutical composition according to (C1), for administration to a patient having renal anemia.
(C6) The pharmaceutical composition according to any one of (C1) to (C5), which is an injectable composition.
(C7) The pharmaceutical composition according to any one of (C1) to (C5), wherein the cell population is constructed as a planar or three-dimensional cellular tissue.

The present invention also includes the following fourth aspect (Invention D).
(D1) A method of improving a state where erythropoietin productivity is decreased in a subject, the method comprising administering or implanting the fibroblast according to any one of (A1) to (A4) and (B5), the cell population according to any one of (A5), (A6), and (B6), or the composition according to (C1) to the subject.
(D2) A method of treating a renal disorder in a subject, the method comprising administering or implanting the fibroblast according to any one of (A1) to (A4) and (B5), the cell population according to any one of (A5), (A6), and (B6), or the composition according to (C1) to the subject.
(D3) A method of treating chronic kidney disease (CKD) in a subject, the method comprising administering or implanting the fibroblast according to any one of (A1) to (A4) and (B5), the cell population according to any one of (A5), (A6), and (B6), or the composition according to (C1) to the subject.
(D4) A method of treating renal anemia in a subject, the method comprising administering or implanting the fibroblast according to any one of (A1) to (A4) and (B5), the cell population according to any one of (A5), (A6), and (B6), or the composition according to (C1) to the subject.
(D5) A method of administering EPO to a subject, the method comprising administering or implanting fibroblasts having enhanced EPO productivity to the subject.

The present invention also includes the following fifth aspect (Invention E).
(E1) Use of the fibroblast according to any one of (A1) to (A4) and (B5), the cell population according to any one of (A5), (A6), and (B6), or the composition according to (C1), as an injection solution for administration to a kidney tissue or a surrounding area thereof.
(E2) Use of the fibroblast according to any one of (A1) to (A4) and (B5), the cell population according to any one of (A5), (A6), and (B6), or the composition according to (C1), as an implantation material for implantation to a kidney tissue or a surrounding area thereof.

### EFFECT OF THE INVENTION

By the present invention, fibroblasts having enhanced EPO productivity were successfully provided. Further, means for improving a state where EPO productivity is decreased, and means for treating a renal disorder or renal anemia, by use of the fibroblasts were successfully provided.

### BRIEF DESCRIPTION OF THE DRAWINGS

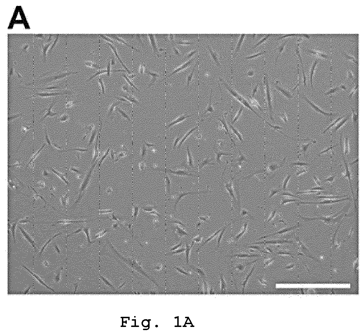

Fig. 1A shows a diagram illustrating characteristics of kidney fibroblasts. Panel A shows a micrographic diagram of cultured kidney fibroblasts (scale bar: 500 µm).

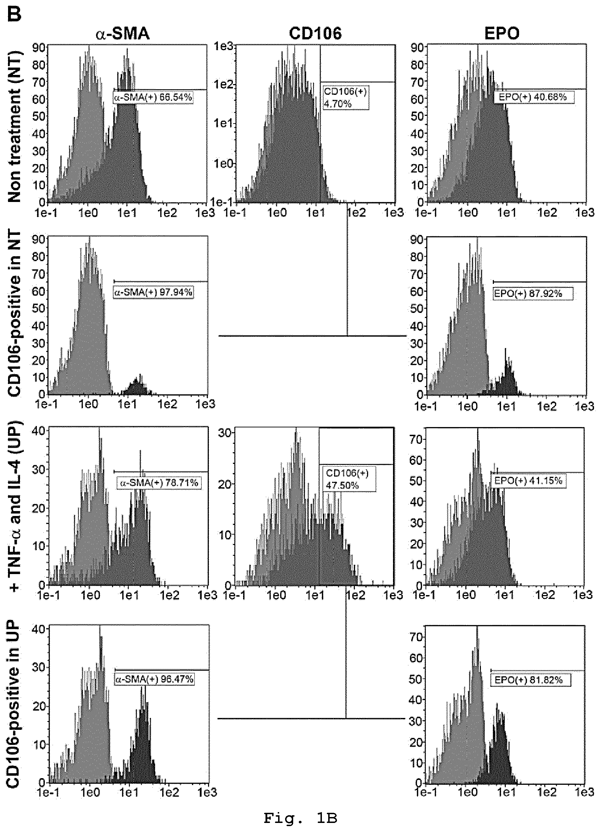

Fig. 1B shows a diagram illustrating characteristics of kidney fibroblasts. Panel B shows flow cytometry analysis diagrams showing expression of actin alpha 2, smooth muscle (a-SMA), CD106, and EPO. The CD106-positive area in untreated cells (Non Treatment (NT)) or cells to which TNF-α and IL-4 were added (+ TNF-a and IL-4 (UP)) was gated, and the cells after the gating were provided as CD106-positive NT (CD 106-positive in NT) cells or CD106-positive UP (CD106-positive in UP) cells, respectively. Each gray histogram shows the fluorescence intensity by an isotype control antibody as a negative control, and each black histogram shows the fluorescence intensity by a labeled antibody against α-SMA, CD106, or EPO.

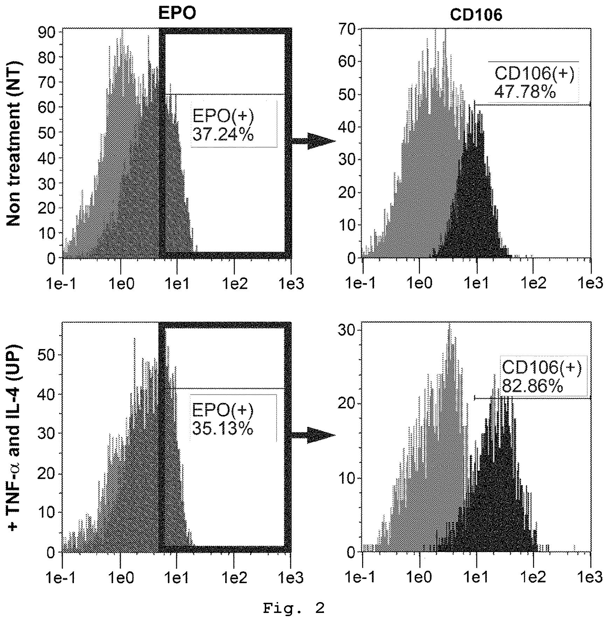

Fig. 2 shows flow cytometry analysis diagrams obtained by gating the EPO-positive area in untreated cells (Non Treatment (NT)) or cells to which TNF-α and IL-4 were added (+ TNF-a and IL-4 (UP)), and investigating the CD106 positive rate in the cells after the gating. Each gray histogram shows the fluorescence intensity by an isotype control antibody as a negative control, and each black histogram shows the fluorescence intensity by a labeled antibody against CD106 or EPO.

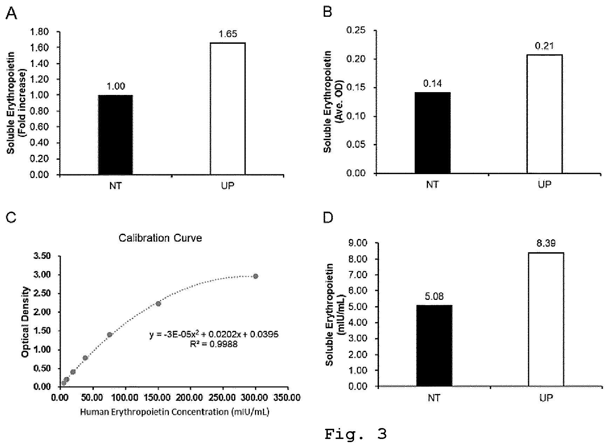

Fig. 3 shows diagrams illustrating relative comparison of the EPO content in a culture supernatant of various fibroblasts. Panel A shows the EPO production in cells to which TNF-α and IL-4 were added (UP), represented as a value relative to the EPO production in untreated cells (Non Treatment (NT)), which is taken as 1. Panel B shows the EPO content in the culture supernatant of various fibroblasts (average optical density; Average OD). Panel C shows a calibration curve. Panel D shows the EPO content in the culture supernatant of various fibroblasts (mIU/mL).

### MODE FOR CARRYING OUT THE INVENTION

The present invention is described below in detail by way of specific embodiments. However, the present invention is not limited to the specific embodiments described.

One embodiment of the present invention is a fibroblast having enhanced EPO productivity.

The fibroblast of the present embodiment is preferably positive for CD106. CD106, also called VCAM-1, is a protein known as a cell adhesion molecule expressed in vascular endothelial cells and the like.

The origin of the fibroblast is not limited. The fibroblast used may be obtained by differentiation from a pluripotent stem cell such as an embryonic stem cell (ES cell), an induced pluripotent stem cell (iPS cell), or a Muse cell, or from an adult stem cell such as a mesenchymal stem cell. Further, a primary cell collected from an animal (including a human) may be used, or a cell line may be used. The fibroblast is preferably derived from the kidney.

In human-derived fibroblasts, the ratio of CD106-positive fibroblasts is low. In the natural state, the ratio is at most about 4.7% (in terms of the cell number) in kidney-derived fibroblasts.

Thus, a human-derived fibroblast population whose ratio of human-derived CD106-positive fibroblasts is increased by the later-mentioned production method may also be included in the present embodiment. For example, another mode of the present invention may be a human-derived fibroblast population whose ratio of human-derived CD106-positive fibroblasts in the total human-derived fibroblasts (in terms of the cell number) is not less than 4.7%. The ratio of CD106-positive fibroblasts in the total fibroblasts in the human-derived fibroblast population of the present embodiment (in terms of the cell number) may be not less than 5%, may be not less than 10%, may be not less than 15%, may be not less than 20%, may be not less than 25%, may be not less than 30%, may be not less than 40%, may be not less than 50%, may be not less than 60%, may be not less than 70%, may be not less than 80%, may be not less than 90%, or may be 100%. By selecting cells known to be positive for CD106, the process of cell sorting can be omitted.

In the natural state, the ratio of EPO-positive (hereinafter also referred to as EPO+) cells in human-derived fibroblasts is low. The ratio is at most about 40.7% (in terms of the cell number) in kidney-derived fibroblasts. Further, since EPO is a hematopoietic hormone rather than a cell membrane-expressed protein, selection or concentration of cells using EPO as an index is difficult.

Thus, for example, by selecting or concentrating human-derived CD106-positive fibroblasts according to the later-mentioned production method for fibroblasts, the ratio of EPO+ fibroblasts in a fibroblast population can be increased. The ratio of EPO+ fibroblasts in a fibroblast population may be increased also by another method. The ratio of EPO+ fibroblasts in the total fibroblasts in the human-derived fibroblast population of the present embodiment (in terms of the cell number) may be not less than 40.7%, may be not less than 45%, may be not less than 50%, may be not less than 60%, may be not less than 70%, may be not less than 80%, may be not less than 90%, or may be 100%.

The enhancement of EPO productivity means, for example, an increase in the EPO production compared to the EPO production in control fibroblasts. The control fibroblasts are not limited, and may be natural kidney fibroblasts. The measurement method for comparison of the EPO production is not limited. For example, flow cytometry, which is a method known to those skilled in the art, may be used to measure the ratio of EPO-positive cells, or the concentration of EPO produced may be measured by the ELISA method.

In cases where flow cytometry is used, the enhancement of the EPO productivity may be judged to have occurred when, for example, the ratio of EPO+ fibroblasts in the total fibroblasts in the human-derived fibroblast population of the present embodiment (in terms of the cell number) is not less than 40.7%, not less than 45%, not less than 50%, not less than 60%, not less than 70%, not less than 80%, not less than 90%, or 100%.

In cases where the ELISA method is used, the enhancement of the EPO productivity may be judged to have occurred when, for example, the amount of EPO contained in the culture supernatant of the human-derived fibroblast population of the present embodiment is increased not less than 1.65-fold, not less than 2.0-fold, not less than 3.0-fold, not less than 5.0-fold, or not less than 10.0-fold compared to control fibroblasts cultured under the same conditions.

Further, for example, after culturing 9.2×10⁵ cells of fibroblasts for 5 days, collecting 3 mL of the culture supernatant, and then concentrating the collected culture supernatant 100-fold using Amicon-Ultra-2, the enhancement of the EPO productivity may be judged to have occurred when the EPO concentration in the culture supernatant concentrate is more than 5.08 mIU/mL, or the EPO productivity may judged to have occurred when the EPO concentration in the culture supernatant concentrate is not less than 5.10 mIU/mL, not less than 6.00 mIU/mL, not less than 7.00 mIU/mL, not less than 8.00 mIU/mL, not less than 8.39 mIU/mL, not less than 10.0 mIU/mL, not less than 20.0 mIU/mL, not less than 50.0 mIU/mL, or not less than 100.0 mIU/mL.

Further, for example, after culturing 9.2×10⁵ cells of fibroblasts for 5 days and collecting 3 mL of the culture supernatant, the enhancement of the EPO productivity may be judged to have occurred when the EPO concentration in the unconcentrated culture supernatant is more than 5.08E-02 mIU/mL, or the EPO productivity may judged to have occurred when the EPO concentration in the unconcentrated culture supernatant is not less than 5.10E-02 mIU/mL, not less than 6.00E-02 mIU/mL, not less than 7.00E-02 mIU/mL, not less than 8.00E-02 mIU/mL, not less than 8.39E-02 mIU/mL, not less than 10.0E-02 mIU/mL, not less than 20.0E-02 mIU/mL, not less than 50.0E-02 mIU/mL, or not less than 100.0E-02 mIU/mL.

Further, for example, after culturing 9.2×10⁵ cells of fibroblasts for 5 days and collecting 3 mL of the culture supernatant, the enhancement of the EPO productivity may be judged to have occurred when the amount of EPO in the unconcentrated culture supernatant per dish (3 mL) is more than 1.52E-01 mIU, or the enhancement of the EPO productivity may be judged to have occurred when the amount of EPO in the unconcentrated culture supernatant per dish (3 mL) is not less than 1.60E-01 mIU, not less than 2.00E-01 mIU, not less than 2.52E-01 mIU, not less than 3.00E-01 mIU, not less than 4.00E-01 mIU, not less than 5.00E-01 mIU, not less than 10.0E-01 mIU, not less than 20.0E-01 mIU, or not less than 50.0E-01 mIU.

Further, for example, after culturing 9.2×10⁵ cells of fibroblasts for 5 days and collecting 3 mL of the culture supernatant, the enhancement of the EPO productivity may be judged to have occurred when the EPO production per cell in the unconcentrated culture supernatant is more than 1.66E-07 mIU, or the EPO productivity may judged to have occurred when the EPO production per cell in the unconcentrated culture supernatant is not less than 1.70E-07 mIU, not less than 1.80E-07 mIU, not less than 1.90E-07 mIU, not less than 2.00E-07 mIU, not less than 2.74E-07 mIU, not less than 3.00E-07 mIU, not less than 5.00E-07 mIU, not less than 10.0E-07 mIU, or not less than 20.0E-07 mIU.

The present embodiment may be a cell population of fibroblast comprising a fibroblast having enhanced EPO productivity. The fibroblast is preferably positive for CD106.

The cell population of the present embodiment may comprise another fibroblast or another component in addition to the CD106-positive fibroblast. In cases where the other fibroblast is contained, the ratio of CD106-positive fibroblasts to the total amount of cells in the cell population in terms of the cell number may be not less than 0.03%, may be not less than 0.1%, may be not less than 1%, may be not less than 2%, may be not less than 4%, may be not less than 5%, may be not less than 10%, may be not less than 20%, may be not less than 30%, may be not less than 40%, may be not less than 50%, may be not less than 60%, may be not less than 70%, may be not less than 80%, may be not less than 90%, may be not less than 95%, may be not less than 98%, or may be not less than 99%

The production method for fibroblasts is described. For the production of the fibroblasts, one may refer to a past report by the present inventors (Patent Document 1), and the production may be carried out according to this report.

### (Preparation of Fibroblasts)

As described above, in the preparation of the fibroblasts, the origin of the fibroblasts is not limited. The fibroblasts may be autologous cells. For example, kidney fibroblasts isolated from a kidney tissue of the patient with a kidney disease, or kidney fibroblasts isolated after allowing differentiation of adult (somatic) stem cells of the patient, are prepared. The fibroblasts may also be fibroblasts collected after allowing differentiation of pluripotent stem cells such as iPS cells derived from autologous cells. The fibroblasts may also be non-autologous cells. For example, a kidney tissue derived from a donor who provides kidney cells, kidney fibroblasts isolated from a kidney tissue prepared using an animal or the like, or kidney fibroblasts isolated after allowing differentiation of adult (somatic) stem cells of a donor, is/are prepared. The fibroblasts may also be fibroblasts collected after allowing differentiation of pluripotent stem cells such as iPS cells or ES cells derived from a donor.

### (Separation of Fibroblasts)

Typically, the fibroblasts prepared can be separated by treatment with an enzyme such as dispase or collagenase. The separation may be carried out by the enzyme such as dispase or collagenase, or may be carried out by another treatment such as mechanical treatment as long as the treatment allows the necessary separation.

### (Selection or Concentration of CD106-Positive Fibroblasts)

In cases where the fibroblasts are positive for CD106, the fibroblasts may be subjected to selection or concentration of CD 106-positive fibroblasts. In cases where CD106-positive fibroblasts can be obtained even without carrying out the selection or concentration, the selection or concentration may be omitted. Examples of the method of the selection or concentration of the CD106-positive fibroblasts include the flow cytometry method, the magnetic bead method, the affinity column method, the panning method, and the like using an anti-CD106-antibody (anti-VCAM-1 antibody). More specifically, the magnetic cell sorting method (MACS), the fluorescence-activated cell sorting method (FACS), or the like may be employed. The anti-CD 106 antibody used may be a commercially available product, or may be prepared by a known method. Either a monoclonal antibody or a polyclonal antibody may be used. From the viewpoint of the specificity, a monoclonal antibody is preferably used. The CD106-positive fibroblasts may also be obtained by removing CD106-negative fibroblasts by introduction of a drug resistance gene. The CD106-positive fibroblasts may also be obtained by introducing a gene encoding a fluorescent protein and isolating fluorescent-protein-positive cells using FACS or the like. The CD106-positive fibroblasts may also be obtained by confirming expression of the CD106 gene in cells using a method such as real-time PCR, nextgeneration sequencer, or in situ hybridization, and isolating a CD106 gene-expressing group.

### (Culture of Fibroblasts)

The fibroblasts may be subjected to culture for the purpose of, for example, achieving a desired cell number, and/or allowing the cells to acquire a desired function. The culture conditions are not limited, and the culture may be carried out by a known cell culture method.

The medium used for the culture may be appropriately set according to, for example, the type of the cells to be cultured. For example, DMEM, α-MEM, RPMI-1640, HFDM-1(+), or the like may be used. The medium may be supplemented with nutritional substances such as FCS and FBS; growth factors; cytokines; antibiotics; and the like. Further, the culture may be carried out in a medium containing TNF-α and/or IL-4.

The number of days of the culture period may be appropriately set for the purpose of, for example, allowing the cultured cells to achieve a desired cell number, and/or allowing the cultured cells to acquire a desired function. Examples of the culture period include periods such as 1 day, 2 days, 3 days, 4 days, 5 days, 6 days, 7 days, 8 days, 9 days, 10 days, 2 weeks, 1 month, 2 months, 3 months, and 6 months.

The culture temperature may be appropriately set according to the type of the cells to be cultured. The culture temperature may be, for example, not less than 10°C, not less than 15°C, not less than 20°C, not less than 25°C, or not less than 30°C, and may be, for example, not more than 60°C, not more than 55°C, not more than 50°C, not more than 45°C, or not more than 40°C.

The culture may be carried out a plurality of times. For example, the culture may be carried out each time when the purity of the desired fibroblasts is increased by selection or concentration.

### (Collection of Fibroblasts)

The cultured fibroblasts may be detached by a protease such as trypsin, and then collected. Alternatively, a temperature-responsive culture dish may be used. In this case, the cells may be detached by changing temperature, and then collected. Alternatively, the cells may be detached using a tool such as a cell scraper, and then collected.

Another mode of the present invention is a method of producing fibroblasts having enhanced EPO productivity, the method comprising: bringing fibroblasts into contact with TNF-α and IL-4; and culturing, under conditions allowing enhancement of EPO productivity, the fibroblasts that have been brought into contact.

The fibroblasts are preferably positive for CD106. In this case, the production method of the present embodiment may comprise selecting or concentrating CD106-positive fibroblasts using an anti-CD106 antibody.

The method of bringing the fibroblasts into contact with TNF-α and IL-4 is not limited as long as the induction of the fibroblasts having enhanced EPO productivity or the induction of the CD106-positive fibroblasts showing enhanced EPO production is not remarkably inhibited. The method is typically, but not necessarily, addition of TNF-α and IL-4 to the medium containing the fibroblasts. TNF-α and IL-4 may be brought into contact with the fibroblasts separately or at the same time. In the method of bringing TNF-α and IL-4 into contact with the fibroblasts at the same time, TNF-α and IL-4 may be mixed together, and then the resulting mixture may be added to the fibroblasts. For example, TNF-α and IL-4 may be dissolved in water, a medium, serum, or dimethyl sulfoxide (DMSO), and then the resulting solution may be added to the medium.

In the present embodiment, the fibroblasts can be cultured in a medium supplemented with TNF-α and IL-4. Therefore, the fibroblast can be cultured while the CD106 expression level in the fibroblasts is maintained. Thus, fibroblasts having a high CD106 expression level can be produced.

In cases where TNF-α and IL-4 are added to the medium (mL), the amount of TNF-α added is not limited. The amount is usually not less than 0.1 ng/mL, may be not less than 0.5 ng/mL, may be not less than 1 ng/mL, may be not less than 10 ng/mL, or may be not less than 50 ng/mL. There is no upper limit of the amount, and the amount is usually not more than 500 ng/mL, or may be not more than 100 ng/mL.

The amount of IL-4 added is not limited. The amount is usually not less than 0.1 ng/mL, may be not less than 0.5 ng/mL, may be not less than 1 ng/mL, or may be not less than 2 ng/mL. There is no upper limit of the amount, and the amount is usually not more than 10 ng/mL, may be not more than 5 ng/mL, or may be not more than 1 ng/mL.

The ratio (weight ratio) between the TNF-α and the IL-4 added, in terms of TNF-a:IL-4, is usually 10,000:1 to 1:1, or may be 50,000:1 to 10:1. Further, the ratio may be 1:1 to 1:10,000, or may be 1:10 to 1:50,000.

By further culturing the fibroblasts after being brought into contact with TNF-α and IL-4, fibroblasts having enhanced EPO productivity can be produced.

The conditions for the culture of the fibroblasts are not limited as long as the conditions allow enhancement of the EPO productivity of the fibroblasts. The culture may be carried out by a known cell culture method. Further, the conditions preferably allow the fibroblasts to become positive for CD106. Examples of the culture method include the method described in the section of (Culture of Fibroblasts) in the above-described production method for fibroblasts.

Examples of the method of collection of the cultured fibroblasts include the method described in the section of (Collection of Fibroblasts) in the above-described production method for fibroblasts.

In cases where the fibroblasts are positive for CD106, the selection or concentration of the CD106-positive fibroblasts may be carried out by a method using an anti-CD 106 antibody. The selection or concentration may be carried out at any timing. The selection or concentration may be carried out before the contact with TNF-α and IL-4, may be carried out after the contact with TNF-α and IL-4 but before the culture, or may be carried out after the culture.

Examples of the selection or concentration include the method described in the section of (Selection and Concentration of CD106-Positive Fibroblasts) in the above-described production method for fibroblasts.

By the selection or concentration using the anti-CD106 antibody, the ratio of CD106-positive fibroblasts in the fibroblasts can be increased to obtain a cell population containing CD106-positive fibroblasts which have enhanced EPO productivity, and which are highly effective for treatment of a disease.

By the method of producing fibroblasts having enhanced EPO productivity of the present embodiment, fibroblasts having enhanced EPO productivity can be obtained. Another embodiment of the present invention is the fibroblasts, or a fibroblast population comprising those fibroblasts. Fibroblasts, especially kidney fibroblasts, exhibit an increased ratio of CD106-expressing cells (in terms of the cell number), and an increased ratio of EPO-expressing cells (in terms of the cell number) when they are stimulated with TNF-α and IL-4. Therefore, fibroblasts having enhanced EPO productivity can be used as a therapeutic composition for a disease that shows a decreased EPO production.

Another embodiment of the present invention is a pharmaceutical composition comprising the fibroblasts having enhanced EPO productivity, or a cell population thereof. The fibroblasts are preferably positive for CD106.

By administering or implanting the pharmaceutical composition of the present embodiment into a living body, highly efficient production of EPO in the living body can be achieved. Therefore, a state where the EPO productivity is decreased due to disorder, deficiency, dysfunction, or the like of a kidney tissue can be improved although the cause of the decrease is not limited thereto. Further, the pharmaceutical composition may be a pharmaceutical composition which may be administered to a patient with a renal disorder or renal anemia, and which is capable of treatment of the renal disorder or renal anemia by the administration. Examples of the renal disorder include, but are not limited to, acute kidney injury (AKI), chronic kidney disease (CKD), diabetic nephropathy, acute glomerulonephritis (acute nephritis), chronic glomerulonephritis (chronic nephritis), acute progressive glomerulonephritis, nephrotic syndrome, acute renal failure, and chronic renal failure. The renal disorder is preferably chronic kidney disease (CKD). The renal disorder is more preferably chronic renal failure. Renal failure means a state where the function of the kidney is decreased compared to that in the normal state. In the present embodiment, in particular, the renal failure means a state where the function of the kidney is decreased to not more than 30% compared to that in the normal state.

The method of the administration or implantation of the pharmaceutical composition of the present embodiment is not limited. For example, the pharmaceutical composition may be administered by injection. The administration site of the pharmaceutical composition is not limited. For example, by directly administering or implanting the pharmaceutical composition into a kidney tissue and/or a surrounding area thereof (such as the subrenal capsule), improvement of the above-described disease state or treatment of renal anemia is possible. The pharmaceutical composition may be administered into the vein, artery, lymph node, lymphatic vessel, bone marrow, subcutaneous tissue, corpus cavernosum penis, or abdominal cavity. The administration into the vein, artery, or lymphatic vessel may be carried out by injection into the vessel, infusion via a catheter, or another known method.

The method of the injection is not limited, and a known injection method such as needle injection or needle-free injection may be applied thereto. The method of the infusion via a catheter is also not limited, and a known method may be applied thereto.

The pharmaceutical composition of the present embodiment may be, but does not necessarily need to be, applied as an artificial organ material to a living body. The artificial organ material may be provided as, for example, a planar or three-dimensional cellular tissue constructed with a cell population. The planar or three-dimensional cellular tissue may be prepared by culturing the fibroblast population alone, or may be prepared by co-culturing the fibroblast population with other cells such as kidney cells and forming the planar or three-dimensional cellular tissue thereafter. Examples of the planar or three-dimensional cellular tissue include, but are not limited to, cell sheets and cell fibers; and tissues formed by a 3D printer.

In the present embodiment, the size and shape of the planar or three-dimensional cellular tissue are not limited as long as the tissue can be applied to a necrotic kidney tissue and/or a surrounding area thereof. The planar tissue may be either a monolayer tissue or multilayer tissue.

The conditions for the cell culture, such as the medium composition, temperature, and humidity, are not limited as long as fibroblasts having enhanced EPO productivity can be cultured, and as long as a planar or three-dimensional cellular tissue can be constructed. Examples of the culture conditions include conditions in accordance with the culture conditions described in the section of (Culture of Fibroblasts) and/or the section of (Collection of Fibroblasts) in the above-described production method for fibroblasts.

By application of such a planar or three-dimensional cellular tissue to a necrotic kidney tissue and/or a surrounding area thereof, or by using such a planar or three-dimensional cellular tissue as an artificial organ for replacement of a necrotic kidney tissue and/or a surrounding area thereof, a renal disorder or renal anemia can be treated.

The ratio of the fibroblasts having enhanced EPO productivity contained in the pharmaceutical composition may be appropriately set based on, for example, the mode of administration or implantation into the patient such that a therapeutically effective amount of the fibroblasts having enhanced EPO productivity are contained as an effective component.

In cases where other fibroblasts are contained in the pharmaceutical composition, the ratio of the fibroblasts having enhanced EPO productivity to the total amount of fibroblasts contained in the pharmaceutical composition in terms of the cell number may be not less than 0.03%, may be not less than 0.1%, may be not less than 1%, may be not less than 2%, may be not less than 4%, may be not less than 5%, may be not less than 10%, may be not less than 20%, may be not less than 30%, may be not less than 40%, may be not less than 50%, may be not less than 60%, may be not less than 70%, may be not less than 80%, may be not less than 90%, may be not less than 95%, may be not less than 98%, or may be not less than 99%.

The number of fibroblasts having enhanced EPO productivity contained in the pharmaceutical composition may be, for example, not less than 1.0×10² cells/mL, not less than 1.0×10³ cells/mL, not less than 1.0×10⁴ cells/mL, not less than 1.0×10⁵ cells/mL, not less than 1.0×10⁶ cells/mL, not less than 5.0×10⁶ cells/mL, or not less than 1.0×10⁷ cells/mL. The number of fibroblasts having enhanced EPO productivity contained in the injectable composition may be further increased or decreased depending on severity of the disease.

The method of confirming the effect of the treatment and/or the improvement by the pharmaceutical composition is not limited, and examples of the method include confirmation based on an increased blood EPO level after the administration or implantation of the composition relative to the level before the administration or implantation of the composition, and confirmation based on an increased number of erythrocyte after the administration or implantation of the composition relative to the number of erythrocyte before the administration or implantation of the composition.

The method of confirming the effect of the treatment and/or the improvement by the pharmaceutical composition is not limited, and examples of the method include confirmation based on a decreased blood creatinine (Cr) level after the administration or implantation of the composition relative to the level before the administration or implantation of the composition, and confirmation based on a decreased urea nitrogen (BUN) value after the administration or implantation of the composition relative to the value before the administration or implantation of the composition.

The method of confirming the effect of the treatment and/or the improvement by the pharmaceutical composition is not limited, and examples of the method include confirmation based on an increased blood creatinine clearance (CCr) value after the administration or implantation of the composition relative to the value before the administration or implantation of the composition, and confirmation based on an increased glomerular filtration rate (GFR) value relative to the value before the administration or implantation of the composition.

The method of confirming the effect of the treatment and/or the improvement by the pharmaceutical composition is not limited, and examples of the method include confirmation based on a decreased level of urinary protein in urine after the administration or implantation of the composition relative to the level before the administration or implantation of the composition, and confirmation based on a decreased level of urinary occult blood relative to the level before the administration or implantation of the composition.

The pharmaceutical composition may also contain other components that are physiologically acceptable as a pharmaceutical composition. Examples of such other components include physiological saline, cell preservation liquids, cell culture media, hydrogels, extracellular matrices, and cryopreservation liquids.

Another embodiment of the present invention may be a method of improving a state where the EPO productivity is decreased, wherein the fibroblasts having enhanced EPO productivity, a cell population thereof, or a pharmaceutical composition comprising these is/are administered or implanted into a subject having the state where the EPO productivity is decreased. The fibroblasts in the present embodiment are preferably positive for CD106.

Another embodiment of the present invention may be a method of treating a renal disorder or protecting the kidney, wherein the fibroblasts having enhanced EPO productivity, a cell population thereof, or a pharmaceutical composition comprising these is/are administered or implanted into a subject having the renal disorder. The fibroblasts in the present embodiment are preferably positive for CD106.

Another embodiment of the present invention may be a method of treating renal anemia, wherein the fibroblasts having enhanced EPO productivity, a cell population thereof, or a pharmaceutical composition comprising these is/are administered or implanted into a subject having the renal anemia. The fibroblasts in the present embodiment are preferably positive for CD106.

Another embodiment of the present invention may be use of the fibroblasts having enhanced EPO productivity, a cell population thereof, or a pharmaceutical composition comprising these, as an injection solution or an implantation material to be administered or implanted into a kidney tissue and/or a surrounding area thereof. The fibroblasts in the present embodiment are preferably positive for CD106.

### EXAMPLES

The present invention is described below in more detail by way of Examples. Needless to say, however, the scope of the present invention is not limited to the Examples.

### <Culture of Human Adult Kidney Fibroblasts>

Human adult kidney fibroblasts were purchased from Innoprot. The cells were cultured in a serum-free medium for fibroblasts (HFDM-1(+), Cell Science & Technology), which medium is supplemented with 1% newborn calf serum (NBCS, Hyclone) and contains a recombinant human epidermal growth factor (rh-EGF). After three to five times of subculture, the fibroblasts were treated with 50 ng/mL TNF-α (Pepro tech) and 2 ng/mL IL-4 (Wako).

### <Flow Cytometry Analysis>

Flow cytometry analysis was carried out using the antibodies and the reagents shown in Table 1. The cells were fixed in 4% paraformaldehyde (Wako), and then left to stand in PBS (Wako). Permeabilization treatment was carried out with 0.1% saponin, and the cells were reacted with the antibodies for 30 minutes each. In the flow cytometry analysis, analysis by MACSQuant Analyzer 10 was carried out in accordance with the instruction provided by the manufacturer (Miltenyi Biotec).

### [Table 1]

**Table 1. Antibodies and reagents used for flowcytometry analysis.**

| Product | Supplier | Reference |
|---|---|---|
| Human alpha-Smooth Muscle Actin APCconjugated Antibody | R&D Systems | IC1420A |
| Alexa Fluor 647 Mouse IgG2, k Isotype Control | BD Biosciences | 557715 |
| BV421 Mouse Anti-human CD106 | BD Biosciences | 744309 |
| BV421 Mouse IgG1, k Isotype Control | BD Biosciences | 562438 |
| APC Human Erythropoietin Ab | Assaypro | 33312-05161 |
| Alexa Fluor 647 Donkey anti-rabbit IgG | Biolegend | 406414 |
| Saponin EP 25G | Nacalai tesque | 30502-42 |
| 4% Paraformaldehyde Phosphate Buffer Solution | Wako | 163-20145 |

### <Evaluation of Erythropoietin Productivity>

Untreated human kidney fibroblasts (NT), or human kidney fibroblasts treated with TNF-α (50 ng/mL) and IL-4 (2 ng/mL) for 3 days (UP), were plated on a culture dish having a diameter of 60 mm (Corning, Corning, NY) at a density of 9.2×10⁵ cells/dish, and culture was carried out for 5 days in serum-free DMEM medium, followed by collection of 3 mL of the culture supernatant of each type of fibroblasts. From each supernatant, a sample was prepared using Amicon-Ultra-2 (Merck, Darmstadt, Germany) and Human Erythropoietin ELISA Kit (Proteintech, Rosemont, IL) according to a protocol recommended by the manufacturer. Measurement was carried out using SpectraMax M2 (San Jose, CA). The EPO production (mIU) before the concentration by Amicon-Ultra-2 was calculated using as a standard the minimum concentration volume (15 µL) described in the data sheet of the product. More specifically, the EPO production (mIU/mL) after the concentration was divided by the concentration rate calculated based on the minimum volume (100-fold; assuming the concentration rate in the case of concentration from the packed volume 1.5 mL to 15 µL), and then multiplied by the total medium volume per dish (3 mL), to calculate the EPO production (mIU). The EPO production per cell (mIU/cell) was calculated by dividing the EPO production (mIU) by the number of plated cells (9.2×10⁵ cells).

### <Example 1: Enhancement of EPO Expression in CD106 (VCAM-1)-Positive Kidney Fibroblasts by Combined Use of TNF-α and IL-4>

Human adult kidney fibroblasts were cultured in a cell culture dish. The human adult kidney fibroblasts had a planar spindle shape, which is a typical shape of fibroblasts (Fig. 1A).

The kidney fibroblasts were treated with 50 ng/mL TNF-α and 2 ng/mL IL-4 for 3 days, and then characteristics of the cells were analyzed using FACS. Antibodies against α-SMA, CD 106, or EPO were used. Staining of protein localized in the cytoplasm was carried out after cell membrane permeabilization treatment with 0.1% saponin.

In the untreated kidney fibroblasts, expression of the fibroblast marker α-SMA was found in 66.54% of the cells. On the other hand, in the kidney fibroblasts, the ratio of CD106-positive cells was small (4.70% in terms of the cell number), and the ratio of EPO+ cells was also small (40.68% in terms of the cell number) (Fig. 1B). As a result of analysis of the CD106-positive cells by gating, the ratios of cells expressing α-SMA or EPO (in terms of the cell number) were 97.94% and 87.92%, respectively. It was thus shown that the ratios of expression of α-SMA and EPO (in terms of the cell number) are high in CD106-positive kidney fibroblasts. From this result, it was shown that, in the natural state, efficient selection or concentration of EPO+ cells by a known method using an anti-CD106 antibody is difficult (Fig. 1B).

Further, by treating the kidney fibroblasts with TNF-α and IL-4 for 3 days, the ratio of CD106-positive cells increased to 47.50% (in terms of the cell number), and the ratio of α-SMA+ cells increased to 78.71% (in terms of the cell number). While the ratio of EPO+ cells in the kidney fibroblasts after the treatment was 41.15% (in terms of the cell number), the ratio of EPO+ cells in the CD106-positive cells was 81.82% (Fig. 1B). It was thus shown that, in CD106-positive kidney fibroblasts, the expression ratios of α-SMA and EPO (in terms of the cell number) are high also after the induction of CD106 by the treatment with TNF-α and IL-4.

### <Example 2: Increase in CD106 Positive Rate in EPO-Expressing Kidney Fibroblasts Due to Combined Use of TNF-α and IL-4>

Human adult kidney fibroblasts were cultured in a cell culture dish. The kidney fibroblasts were treated with 50 ng/mL TNF-α and 2 ng/mL IL-4 for 3 days, and then characteristics of the cells were analyzed using FACS. Antibodies against CD106 or EPO were used.

In the untreated kidney fibroblasts, the ratio of EPO+ cells was 37.24% in terms of the cell number, and, among these, the ratio of CD106 positive cells was 47.78%. On the other hand, in the kidney fibroblasts treated with TNF-α and IL-4 for 3 days, the ratio of EPO+ cells was 35.13% in terms of the cell number, and, among these, the ratio of CD106 positive cells was 82.86%, indicating a remarkably high ratio of CD106 positive cells.

It was thus shown that highly pure CD106-positive fibroblasts having enhanced EPO productivity can be efficiently obtained by purifying the kidney fibroblasts using a known method that uses an anti-CD106 antibody. From these results, it was suggested that, by using TNF-α and IL-4 to selectively impart CD106 positivity to EPO-producing kidney fibroblasts, selection or concentration of CD106-positive fibroblasts having enhanced EPO productivity can be efficiently carried out.

### <Example 3: Evaluation of Erythropoietin Productivity of Various Fibroblasts>

In order to evaluate the erythropoietin productivities of untreated human kidney fibroblasts (NT), and human kidney fibroblasts treated with TNF-α (50 ng/mL) and IL-4 (2 ng/mL) for 3 days (UP), the erythropoietin content in the culture supernatant of various fibroblasts was evaluated by ELISA (Fig. 3). As a result, it was shown that the culture supernatant of UP contains 1.65 times the amount of EPO compared to the culture supernatant of NT (Fig. 3A). The average optical density (Average. OD) of the culture supernatant of UP was 0.21, indicating that EPO is contained at a concentration of 8.39 mIU/mL. On the other hand, the Average. OD of the culture supernatant of NT was 0.14, indicating that 5.08 mIU/mL EPO (after concentration) is contained (Figs. 3B and D). The culture supernatant of UP before the concentration contained EPO at a concentration of 8.39E-02 mIU/mL, 2.52E-01 mIU, or 2.74E-07 mIU/cell. On the other hand, the culture supernatant of NT before the concentration contained EPO at a concentration of 5.08E-02 mIU/mL, 1.52E-01 mIU, or 1.66E-07 mIU/cell.

From the present result, it became clear that kidney fibroblasts prepared by treatment with TNF-α and IL-4 show not only enhanced expression, but also improved productivity of EPO. It was thus suggested that, by using TNF-α and IL-4 to selectively impart CD106 positivity to EPO-producing kidney fibroblasts, selection or concentration of CD106-positive fibroblasts having enhanced EPO productivity can be efficiently carried out.

It is known that EPO is a hormone that contributes to promotion of erythrocyte production. EPO is mostly produced in the kidney, and the produced EPO acts on erythroblastic precursor cells in bone marrow to play a role in promotion of erythrocyte production (differentiation and proliferation). Further, in recent years, erythrocyte production-promoting ability of EPO is drawing attention, and effectiveness of EPO for kidney diseases such as renal anemia has been revealed (Non-patent Document 1). EPO preparations are widely used as therapeutic agents for renal anemia in actual clinical sites. EPO is also known to have anti-inflammatory, antioxidative, antiapoptotic, and angiogenic promoting actions in various tissues. It has also been reported that, by administration of EPO to a subject having a renal disorder (acute kidney injury (AKI), chronic kidney disease (CKD), diabetic nephropathy, or the like), EPO provides a kidney protection function that protects the kidney against a decrease in renal function caused by the renal disorder (Non-patent Documents 2 and 3). Thus, in view of the roles of EPO known from these preceding studies, it is suggested that administration of CD106-positive fibroblasts artificially prepared by the present invention having enhanced EPO productivity is effective for a renal disorder.

## Claims

1. A fibroblast having enhanced erythropoietin (EPO) productivity.

2. The fibroblast according to claim 1, which is positive for CD106.

3. The fibroblast according to claim 1 or 2, wherein the fibroblast is a kidney fibroblast.

4. The fibroblast according to any one of claims 1 to 3, wherein the EPO production is not less than 1.65 times higher than in a control fibroblast.

5. A cell population of fibroblast comprising the fibroblast according to any one of claims 1 to 4.

6. The cell population according to claim 5, wherein the ratio of the CD106-positive fibroblast (in terms of the cell number) is not less than 4.7% relative to the total fibroblasts contained in the cell population.

7. A method of producing fibroblasts having enhanced EPO productivity, the method comprising:
bringing fibroblasts into contact with TNF-α and IL-4; and
culturing, under conditions allowing enhancement of EPO productivity, the fibroblasts that have been brought into contact.

8. The method according to claim 7, wherein the fibroblasts having enhanced EPO productivity are positive for CD106.

9. The method according to claim 8, further comprising selecting or concentrating CD106-positive fibroblasts using an anti-CD 106 antibody.

10. The method according to any one of claims 7 to 9, wherein the fibroblasts are kidney fibroblasts.

11. A fibroblast having enhanced EPO productivity, obtained by the production method according to any one of claims 7 to 10.

12. A cell population of fibroblast comprising the fibroblast according to claim 11.

13. A pharmaceutical composition comprising: the fibroblast according to any one of claims 1 to 4 and 11; or the cell population according to any one of claims 5, 6, and 12.

14. The pharmaceutical composition according to claim 13, for improvement of a state where EPO productivity is decreased.

15. The pharmaceutical composition according to claim 13, for administration to a patient having a renal disorder.

16. The pharmaceutical composition according to claim 15, wherein the renal disorder is chronic kidney disease.

17. The pharmaceutical composition according to claim 13, for administration to a patient having renal anemia.

18. The pharmaceutical composition according to any one of claims 13 to 17, which is an injectable composition.

19. The pharmaceutical composition according to any one of claims 13 to 17, wherein the cell population is constructed as a planar or three-dimensional cellular tissue.
